(19) Europäisches Patentamt
European Patent Office
Office européen des brevets

(11) Numéro de publication: **0 138 684**
**B1**

# (12) FASCICULE DE BREVET EUROPEEN

(45) Date de publication du fascicule du brevet: **12.04.89**

(51) Int. Cl.⁴: **C 07 D 295/12**, C 07 D 317/66, A 61 K 31/40

(21) Numéro de dépôt: **84401919.0**

(22) Date de dépôt: **26.09.84**

(54) **2-(N-pyrrolidino)-3-isobutoxy-N-phenyl substitué N-benzyl propylamines, leur préparation et leur application pharmaceutique.**

(30) Priorité: **27.09.83 FR 8315367**

(43) Date de publication de la demande: **24.04.85 Bulletin 85/17**

(45) Mention de la délivrance du brevet: **12.04.89 Bulletin 89/15**

(84) Etats contractants désignés: **AT BE CH DE FR GB IT LI LU NL SE**

(56) Documents cités:
**FR-A-2 174 655**
**FR-A-2 378 024**

**Le dossier contient des informations techniques présentées postérieurement au dépôt de la demande et ne figurant pas dans le présent fascicule.**

(73) Titulaire: **RIOM LABORATOIRES- C.E.R.M. "R.L.-CERM" - (S.A.), Route de Marsat B.P. 140, F-63203 Riom Cédex (FR)**

(72) Inventeur: **Monteil, André, Route de Prompsat Les Grosliers, F-63140 Chatel-Guyon (FR)**
Inventeur: **Simond, Jacques, Rue des Thuilets Mirefleurs, F-63730 Les-Martres-De-Veyre (FR)**
Inventeur: **Combourieu, Michel, 6, rue du Mont Mouchet, F-15000 Aurillac (FR)**

(74) Mandataire: **Hermans, Franciscus G.M. et al, Patent Department AKZO N.V. Pharma Division P.O. Box 20, NL-5340 BH Oss (NL)**

**EP 0 138 684 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouvelles 2-(N-pyrrolidino)-3-isobutoxy-N-phenyl substitué N-benzyl propylamines, leur préparation et leur application pharmaceutique.

Plus précisément, ces nouveaux dérivés correspondent à la formule générale suivante:

Composés de formule:

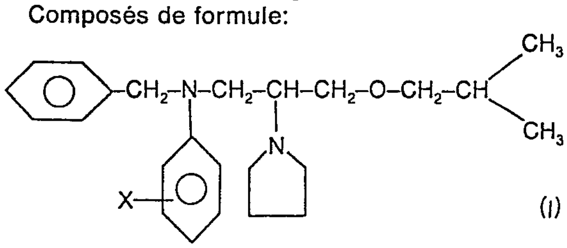

(I)

dans laquelle X représente un ou deux radicaux choisis parmi les radicaux halogéno, hydroxy, alcoxy ayant 1 à 6 atomes de carbone, alkyle linéaire ou ramifié ayant 1 à 6 atomes de carbone, trifluorométhyle ou méthylènedioxy.

L'invention concerne aussi les sels d'addition des composés de formule I avec des acides organiques ou inorganiques pharmaceutiquement acceptables, tels que les acides chlorhydrique, fumarique, maléique, citrique ou succinique, ces acides n'étant mentionnés qu'à titre illustratif mais sans constituer une limitation.

On connaît déjà plusieurs propylamines substituées, tels que les composés du brevet FR A-2 174 655 ayant des substituants comparables aux composés revendiqués, mais en position isomère. Plus récemment, le brevet FR A-2 378 024 décrit une structure de type I à activité antiangoreuse.

Par rapport à ces composés antérieurs, les études pharmacologiques ont montré que les composés de l'invention possédaient d'intéressantes propriétés cardiovasculaires et plus particulièrement des propriétés anti-angineuse, anti-hypertensive, et antidysrythmique; ce profil cardiovasculaire est de façon surprenante plus actif et plus spécifique que celui des composés de l'art antérieur, en particulier sur la consommation d'oxygène tant en intensité qu'en durée d'action.

Parmi les composés de formule I et leurs sels, les composés préférés sont ceux pour lesquels X correspond à une di-substitution, dans laquelle les deux substituants sont de préférence identiques, tels que di-halogéno, di-méthyle, diméthoxy ou méthylènedioxy. Plus particulièrement une réalisation préférée concerne les exemples de substitutions ortho-ortho di-méthyle, méta-para di-halogéno et méta-para méthylènedioxy.

Une autre réalisation préférée de l'invention – lorsque X correspond à une monosubstitution – est représentée par les composés de formule I dans lesquels X est un substituant en position méta ou para. Selon cette réalisation les composés préférés sont ceux dans lesquels X représente un groupe méta-chloro, para-chloro, méta-méthoxy ou para-méthoxy.

Les composés de l'invention peuvent être préparés de différentes façons bien connues pour la préparation de composés analogues.

Un procédé approprié pour la préparation desdits composés consiste en une réaction de condensation d'un composé de formule:

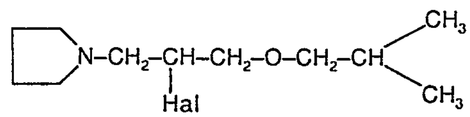

[A]

ou de l'un de ses sels d'addition dans lequel Hal représente un halogène, de préférence le chlore, avec un composé de formule:

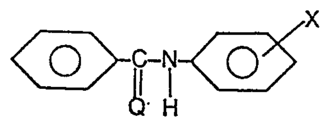

[B]

ou de l'un de ses sels d'addition dans lequel X a la signification précédemment indiquée et Q représente soit l'oxygène, soit deux atomes d'hydrogène.

Si Q représente l'oxygène, le composé carbonyl résultant peut ensuite être réduit au moyen d'un agent d'hydrogénation.

Dans ladite réaction de condensation, on fait de préférence réagir le composé [B] sur le composé [A] en présence d'un composé basique, tel que l'hydroxyde de sodium, ou un composé ammonium quaternaire.

Selon une variante de cette réaction de condensation, le composé de formule [B] subit d'abord une métallation à l'aide d'agents bien connus dans l'art antérieur, tels que l'hydrure de sodium, le sodium, le potassium ou l'amidure de lithium, dans un solvant organique tels que le benzène, le toluène, le xylène, le diméthylformamide ou le diméthylsulfoxyde.

L'éventuelle hydrogénation (si Q représente l'oxygène) est effectuée de préférence au moyen d'un hydrure métallique ou d'un complexe d'hydrure métallique tel que le diborane.

La matière première de formule [B] peut être préparée en faisant réagir

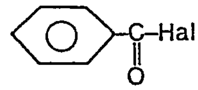 avec 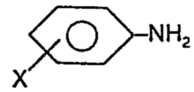

[C] [D]

suivi si nécessaire d'une hydrogénation du composé résultant. Les propriétés cardiovasculaires des composés de l'invention ont été mises en évidence par des expérimentations pharmacologiques effectuées in vitro et in vivo. In vitro, on a recherché l'activité anticalcique conformément aux protocoles résumés ci-après.

Recherche d'un tropisme cardiaque

Dans une solution de KREBS-HENSELEIT maintenue à 37 °C, on place un muscle papillaire de lapin stimulé électriquement à la fréquence de 1,5 Hz (5 ms d'impulsion de 15 V). L'effet inotrope

positif du chlorure de calcium est recherché selon la technique des courbes doses-réponses cumulatives.

Les substances étudiées sont adjointes à la solution de survie 15 minutes avant la réalisation des courbes agonistes.

Recherche d'un tropisme vasculaire

Dans une solution de KREBS maintenue à 37 °C, dépourvue de $Ca^{++}$ et enrichie en $K^{+}$ (6 mg/l de KCl) pour dépolarisation, on place une aorte isolée de lapin découpée en spirales. L'effet contracturant de concentrations cumulatives de chlorure de calcium est recherché et l'effet anticalcique des composés étudiés est évalué 15 minutes après leur adjonction à la solution de survie.

Les paramètres classiques de pharmacologie moléculaire sont ensuite déterminés ($pA_2$ pour un antagonisme compétitif et $pD'_2$ pour un antagonisme non compétitif). Selon la technique de VAN ROSSUM [ARCH. INT. PHARMACODY. THER. 143, 299–330 (1963)].

Ces résultats sont rapportés dans le tableau I ci-après.

Tableau I

| Composé N°* | Muscle papillaire | | Aorte isolée | |
|---|---|---|---|---|
| | $pA_2$ | $pD'_2$ | $pA_2$ | $pD'_2$ |
| 1 | 4,30 ± 0,24 | 3,81 ± 0,20 | 4,83 ± 0,28 | 5,08 ± 0,27 |
| 3 | 4,87 ± 0,40 | 4,5 à $10^{-4}$M | 4,70 ± 0,38 | 4,00 ± 0,24 |
| 5 | Inactif à $10^{-4}$M | / | 4,83 ± 0,27 | 4,71 ± 0,20 |
| 6 | 3,41 à $10^{-4}$M | / | 5,19 ± 0,37 | 3,73 ± 0,50 |
| 7 | Inactif à $10^{-4}$M | / | 4,70 ± 0,60 | 3,85 ± 0,43 |
| 8 | 4,67 à $10^{-5}$M | 3,69 à $10^{-4}$M | 5,21 ± 0,65 | 4,87 ± 0,48 |
| 11 | 5,44 ± 0,26 | Inhibition totale à $10^{-4}$M | 4,94 ± 0,43 | 4,80 ± 0,05 |

* identification des composés dans tableau III

Ces résultats montrent que les composés de l'invention possèdent d'intéressantes propriétés anticalciques, le composé n° 11 étant celui qui possède la plus grande activité tant au niveau cardiaque qu'au niveau vasculaire; l'activité du composé n° 8 est également notable. D'un autre côté, on a trouvé que les composés 1 et 3 possèdent un tropisme cardiaque plus marqué, alors que le composé n° 6 montre un tropisme vasculaire prédominant.

In vivo, on a recherché l'activité antiangineuse en mesurant les effets hémodynamiques chez le chien anesthésié, conformément au protocole résumé ci-après.

Chez le chien anesthésié au chloralose (100 $mg.kg^{-1}$ I.V.), les paramètres suivants sont enregistrés:

– fréquence cardiaque à l'aide d'électrodes à E C G sous-cutanées reliées à un cardiotachymètre BECKMAN (dérivation D II),

– débit artériel coronaire enregistré par l'intermédiaire d'un débitmètre électromagnétique STATHAN,

– action antitachycardisante (inhibition des effets chronotropes positifs de l'isoprénaline).

Ces paramètres sont enregistrés en continu sur un dynographe BECKMAN, et on mesure en même temps la durée d'action. Les composés de l'invention sont administrés par voie I.V. à la dose de 5 $mg.kg^{-1}$.

Les résultats enregistrés, exprimés sous forme de pourcentage de variation, sont résumés dans le tableau II ci-après.

Tableau II

| Composé N°* | Fréquence cardiaque Variation (%) | Durée (mn) | Débit coronaire Variation (%) | Durée (mn) | Action antitachycardisante Variation (%) | Durée (mn) |
|---|---|---|---|---|---|---|
| 2 | −11 | >45 | + 39 | 10 | −51 | 15 |
| 3 | −24 | >45 | +196 | 20 | −63 | 30 |
| 4 | −27 | >45 | + 32 | 3 | −60 | >45 |
| 5 | −20 | >45 | + 98 | 15 | −45 | 15 |
| 6 | −32 | >45 | + 20 | 1 | −64 | >45 |
| 7 | −23 | 45 | + 31 | 5 | −54 | 45 |
| 8 | −36 | 35 | + 85 | 15 | −28 | 15 |
| 11 | −50 | >45 | +116 | 20 | −47 | 45 |

* identification des composés dans tableau III

Ces résultats montrent que les composés de l'invention présentent tous des activités bradycardisantes après administration I.V. à la dose de 5 $mg.kg^{-1}$. D'autre part, la majorité de ces substances possède une action antitachycardisante puissante.

Il apparaît que les composés possédant l'activité antiangineuse la plus intéressante sont les composés n° 11, 8, 2, 3 et 5.

Les composés de l'invention se sont en outre révélés posséder une toxicité réduite: leur toxicité aiguë par voie orale chez la souris est généralement supérieure à 500 $mg.kg^{-1}$.

Cet ensemble de propriétés pharmacologiques met en évidence la possibilité d'application des composés de l'invention en thérapeutique humaine, en tant que médicaments pour le traitement des troubles cardiovasculaires, tels que l'angine de poitrine, l'hypertension ou les troubles du rythme.

Associés aux excipients pharmaceutiques habituels, ils pourront être administrés, par voie orale ou intraveineuse, à des doses quotidiennes comprises entre 1 et 15 mg par kg de poids corporel.

Mélangés avec des excipients convenables, les composés de formule I ou leurs sels peuvent être comprimés sous forme de prises unitaires tels que comprimés, pilules etc... ou peuvent être mis en capsules. Au moyen de liquides convenables, les composés peuvent aussi être utilisés en préparations injectables ou orales sous forme de solutions, suspensions ou émulsions.

Les composés de formule I possèdent au moins un atome de carbone asymétrique, de sorte qu'il est possible d'obtenir un mélange racémique de I et les énantiomères optiques séparés. Le mélange racémique et les énantiomères optiques séparés appartiennent à la fois aux composés de l'invention. Les énantiomères optiques peuvent être séparés de manière habituelle par résolution du mélange racémique, ou directement en partant de matières premières optiquement actives.

Le radical alkyle dans la définition de X est un radical alkyle linéaire ou ramifié de 1 à 6 atomes de carbone, et plus spécialement de 1 à 4 atomes de carbone tels que méthyle, éthyle, propyle, butyle ou isobutyle. Le radical méthyle est le radical préféré.

Le radical alkyle dans le radical alcoxy défini pour X a une signification analogue.

L'halogène dans la définition de X est de préférence le chlore ou le brome.

EXEMPLE 1

2-(N-pyrrolidino)-3-isobutoxy-N-(3,4-méthylènedioxy)phényl N-benzyl propylamine

Dans un réacteur contenant 41 g (0,3 M) de 3,4-méthylènedioxy aniline et 75 ml (0,9 M) de triéthylamine dans 500 ml de toluène, on a ajouté goutte à goutte 42 g (0,3 M) de chlorure de benzoyle à température ambiante, puis on a chauffé pendant 4 heures à 40 °C. Après avoir laissé le milieu réactionnel revenir à température ambiante, on a encore ajouté 20 g de chlorure de benzoyle et 30 ml de triéthylamine et maintenu un chauffage à 40 °C pendant 9 heures. En fin de réaction, on a filtré le précipité formé, lavé avec une solution de carbonate de soude, puis à l'eau. On l'a ensuite repris par du chlorure de méthylène, lavé à l'eau, puis distillé le solvant et séché la phase organique. On a ainsi obtenu 40 g de 3',4'-méthylènedioxy benzanilide ayant pour point de fusion F = 136 °C.

Dans une deuxième étape, on a introduit 15 g (0,06 M) de l'amide précédent dans un réacteur contenant 100 ml de soude 10 N et chauffé ce mélange 3 heures à 80 °C, laissé revenir à température ambiante, puis ajouté 1,3 g (0,006 M) de chlorure de benzyle triéthylammonium et 15 g de (2-chloro-3-isobutoxy) propyl-1-pyrrolidine et chauffé le mélange 6 heures à 70 °C. L'amide formé a été extrait au chlorure de méthylène; après séchage de la phase organique et élimination du solvant, on a obtenu par distillation 18 g de N-[2-(N-pyrrolidino)-3-isobutoxy] propyl-3'4'-méthylènedioxy benzanilide

ayant pour point d'ébullition E = 213 °C/ 0,5 mm Hg.

Dans la troisième étape, on a introduit 6 g (0,12 M) de borohydrure de sodium dans 75 ml de tétrahydrofurane puis ajouté goutte à goutte 17 g (0,039 M) de l'amide précédent dissous dans 35 ml de tétrahydrofurane. En maintenant le mélange réactionnel sous courant d'azote, on a ensuite ajouté goutte à goutte 37 ml de trifluorure de bore éthyl éthérate dissous dans 50 ml de tétrahydrofurane et laissé la réaction se dérouler 4 heures sous agitation. Le mélange a ensuite été hydrolysé par addition de 60 ml d'une solution d'acide chlorhydrique 2 N, puis le solvant chassé par distillation. On a alcalinisé le mélange par une solution de soude 2 N, puis extrait la base au chlorure de méthylène, séché la phase organique puis chassé le solvant. Après transformation du produit obtenu en fumarate et recristallisation de ce dernier dans l'éthanol, on a obtenu 13,5 g de produit du titre sous forme de fumarate ayant pour point de fusion F = 124 °C et pour analyse élémentaire:

| | C % | H % | N % |
|---|---|---|---|
| Calculé: | 66,14 | 7,27 | 5,32 |
| Trouvé: | 66,15 | 7,16 | 5,26. |

EXEMPLE 2

2-(N-pyrrolidino)-3-isobutoxy-N-(3-chloro)phényl N-benzyl propylamine

En procédant comme indiqué à l'exemple précédent, on a d'abord préparé le 3'-chloro benzanilide qu'on a ensuite traité comme indiqué à l'exemple 1 par la 1-(2-chloro-3-isobutoxy) propylpyrrolidine pour obtenir le N-[2-(N-pyrrolidino)-3-isobutoxy]propyl-3'-chloro benzanilide. Cet amide a ensuite été réduit: dans un réacteur contenant une suspension de 5,1 g de borohydrure de sodium dans 30 ml de THF, maintenue entre 0° et 5 °C, on a ajouté goutte à goutte une solution de 24 ml de $BF_3Et_2O$ dans 15 ml de THF, puis laissé la réaction se dérouler, à température ambiante, sous courant d'azote en maintenant l'agitation pendant 4 heures. On a ensuite hydrolysé le milieu réactionnel par addition de 100 ml d'acide chlorhydrique à 20% puis de 100 ml d'eau. Après avoir éliminé le solvant organique par distillation, on a alcalinisé à la soude 10 N, puis extrait le composé du titre au chlorure de méthylène.

Après élimination du solvant et séchage, on a transformé le composé en fumarate dans un mélange éthanol + éther et recristallisé dans l'acétate d'éthyle. On a ainsi obtenu 10 g de produit ayant pour point de fusion F = 109 °C et analyse élémentaire:

| | C % | H % | N % |
|---|---|---|---|
| Calculé: | 65,04 | 7,21 | 5,42 |
| Troúvé: | 66,20 | 7,32 | 5,49. |

EXEMPLE 3

2-(N-pyrrolidino)-3-isobutoxy-N-(4-hydroxy)phényl N-benzyl propylamine

En partant de 4-benzyloxy aniline et de chlorure de benzoyle, on a d'abord obtenu le 4'-benzyloxy benzanilide qu'on a fait réagir avec la 1-(2-chloro-3-isobutoxy) propyl-pyrrolidine, pour obtenir le N-[2-(N-pyrrolidino) 3-isobutoxy] propyl-4'-benzyloxy benzanilide.

Ce composé est ensuite débenzylé par hydrogénation catalytique: dans un appareil de Parr contenant 370 ml d'éthanol absolu, on a introduit 3,7 g de catalyseur (Pd/C à 5%) et 37 g de l'amide précédent, puis amené le milieu réactionnel à pH 1 en ajoutant de l'éthanol saturé en HCl, puis on a agité le mélange sous une pression d'hydrogène de 28 kg/cm² pendant 4 jours en changeant deux fois le catalyseur. La réaction terminée, on a filtré le catalyseur et éliminé l'éthanol par distillation. Après avoir alcalinisé à l'ammoniaque, on a extrait la base au chlorure de méthylène pour obtenir 27 g de N-[2-(N-pyrrolidino)-3-isobutoxy] propyl 4'-hydroxy benzanilide.

Dans une dernière étape, on a réduit l'amide obtenu comme indiqué dans les exemples précédents.

En faisant réagir 26 g d'amide, 11 g de $NaBH_4$ et 50 ml de $BF_3Et_2O$ dans le THF, pendant 6 heures à reflux de solvant, puis après hydrolyse à froid par 180 ml d'acide chlorhydrique 6 N, on a obtenu 15,8 g de composé du titre sous forme de dichlorhydrate ayant pour point de fusion F = 131 °C et pour analyse élémentaire:

| | C % | H % | N % |
|---|---|---|---|
| Calculé: | 63,29 | 7,96 | 6,15 |
| Trouvé: | 63,10 | 8,06 | 6,02. |

Les composés des exemples précédents, ainsi que quelques autres composés de l'invention (tous préparés selon l'exemple 1) ont leurs caractéristiques résumées dans le tableau III ci-après.

Tableau III

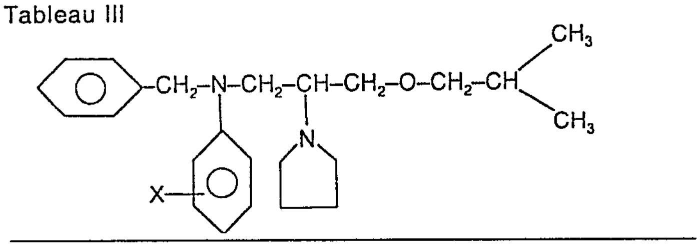

| Composé N° | X | Sel | Point de fusion °C |
|---|---|---|---|
| 1 | 4-$CH_3$ | Fumarate | 127 |
| 2 | 3,4-diCl | / | 68 (base) |
| 3 | 4-$OCH_3$ | Fumarate | 136 |
| 4 | 4-Cl | / | 74 (base) |
| 5** | 3-Cl | Fumarate | 109 |
| 6 | 3-$CF_3$ | HCl | 150 |
| 7 | 3,5-diCl | HCl | 142 |
| 8 | 2,6-di$CH_3$ | HCl | 181 |
| 9 | 2-$OCH_3$ | Fumarate | 104 |
| 10*** | 4-OH | 2 HCl | 131 |
| 11* | 3,4-$OCH_2O$- | Fumarate | 124 |
| 12 | 3-Br | Oxalate | 137,5 |

* Composé de l'exemple 1
** Composé de l'exemple 2
*** Composé de l'exemple 3

## Revendications

1. Composés de formule générale I

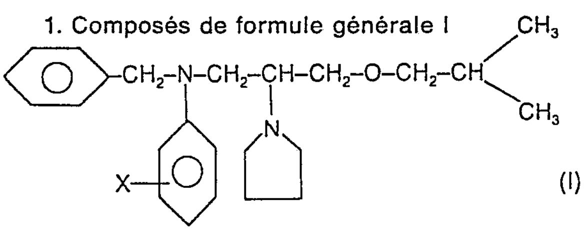

(I)

dans laquelle X représente un ou deux radicaux choisis parmi les radicaux halogéno, hydroxy, alcoxy ayant 1 à 6 atomes de carbone, alkyle linéaire ou ramifié ayant 1 à 6 atomes de carbone, trifluorométhyle ou méthylènedioxy et leurs sels pharmaceutiquement acceptables.

2. Composés selon la revendication 1 caractérisés en ce que X correspond à une disubstitution

dans laquelle les deux substituants sont identiques.

3. Composés selon la revendication 2 caractérisés en ce que X représente une substitution di-halogéno, di-méthyle, di-méthoxy ou méthylènedioxy.

4. Composés selon la revendication 1 caractérisés en ce que X représente une monosubstitution en position méta ou para.

5. Composé selon la revendication 3 caractérisé en ce que X représente le radical 3,4-méthylènedioxy.

6. Composé selon la revendication 3 caractérisé en ce que X représente les radicaux méthyle en position 2 et 6.

7. Composé selon la revendication 3 caractérisé en ce que X représente des atomes de chlore en position 3 et 4.

8. Composé selon la revendication 4 caractérisé en ce que X représente un radical para-méthoxy ou méta-chloro.

9. Procédé pour la préparation d'un composé selon la revendication 1 caractérisé en ce qu'on fait réagir un composé de formule:

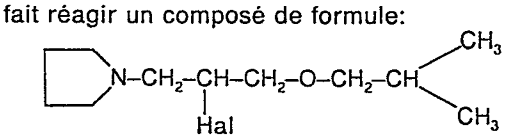
[A]

ou l'un de ses sels d'addition acide avec un composé de formule:

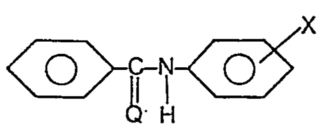
[B]

ou l'un de ses sels d'addition acide dans lequel X a la signification précédemment indiquée, et Q représente soit l'oxygène, soit deux atomes d'hydrogène, et qu'ensuite – lorsque Q représente l'oxygène – on réduit le composé à fonction carbonyl, et qu'éventuellement on le convertit en sel d'addition acide pharmaceutiquement acceptable.

10. Procédé selon la revendication 9 caractérisé en ce que la réaction entre [A] et [B] est facilitée en effectuant préalablement la métallation du composé de formule [B] avec un hydrure ou une amide de métal alcalin.

11. Composition pharmaceutique utile comme médicament pour les troubles cardiovasculaires, spécialement l'angine de poitrine, l'hypertension et les troubles du rythme, caractérisée en ce qu'elle contient comme principe actif au moins un des composés selon l'une des revendications 1 à 8, associé avec des excipients convenables.

12. Composition pharmaceutique selon la revendication 11 caractérisée en ce qu'elle contient une dose de principe actif permettant une administration journalière comprise entre 1 et 15 mg par kg de poids corporel.

## Claims

1. Compounds of the general formula I

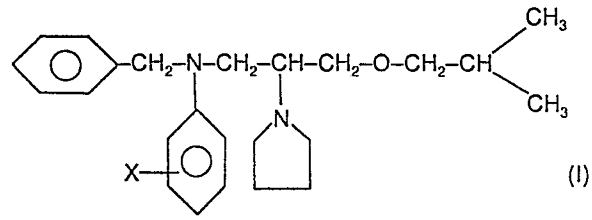
(I)

in which X denotes one or two radicals chosen from halo or hydroxy radicals, alkoxy radicals having 1 to 6 carbon atoms, linear or branched alkyl radicals having 1 to 6 carbon atoms, or trifluoromethyl or methylenedioxy radicals, and their pharmaceutically acceptable salts.

2. Compounds according to Claim 1, characterized in that X corresponds to a disubstitution in which the two substituents are identical.

3. Compounds according to Claim 2, characterized in that X denotes a dihalo, dimethyl, dimethoxy or methylenedioxy substitution.

4. Compounds according to Claim 1, characterized in that X denotes a monosubstitution in the meta- or para- position.

5. Compound according to Claim 3, characterized in that X denotes a 3,4-methylenedioxy radical.

6. Compound according to Claim 3, characterized in that X denotes methyl radicals in the 2- and 6-positions.

7. Compound according to Claim 3, characterized in that X denotes chlorine atoms in the 3- and 4-positions.

8. Compound according to Claim 4, characterized in that X denotes a para-methoxy or meta-chloro radical.

9. Process for preparing a compound according to Claim 1, characterized in that a compound of formula:

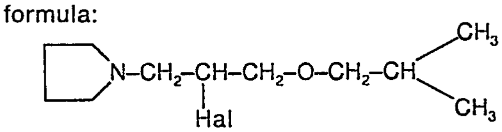
[A]

or one of its addition salts with an acid, is reacted with a compound of formula:

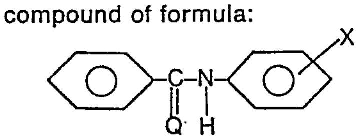
[B]

or one of its addition salts with an acid, in which X has the meaning stated above and Q denotes either oxygen or two hydrogen atoms, and in that – when Q denotes oxygen – the compound containing a carbonyl group is then reduced, and in that it is optionally converted to an addition salt with a pharmaceutically acceptable acid.

10. Process according to Claim 9, characterized in that the reaction between [A] and [B] is facilitated by permorming a prior metallation of the compound of formula [B] with an alkali metal amide or hydride.

11. Pharmaceutical composition which is useful as a medicinal product for cardiovascular disorders, especially Angina pectoris, hypertension and disorders of rhythm, characterized in that it contains, as active principle, at least one of the

compounds according to one of Claims 1 to 8, in combination with suitable excipients.

12. Pharmaceutical composition according to Claim 11, characterized in that it contains a dose of active principle permitting a daily administration of between 1 and 15 mg per kg of body weight.

**Patentansprüche**

1. Verbindungen der allgemeinen Formel I

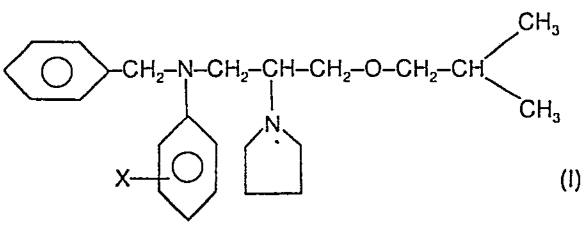

(I)

worin X für einen oder zwei unter Halogen, Hydroxyl, Alkoxy mit 1 bis 6 Kohlenstoffatomen, linearem oder verzweigtem Alkyl mit 1 bis 6 Kohlenstoffatomen, Trifluormethyl oder Methylendioxy ausgewählte Reste steht, sowie deren pharmazeutisch unbedenkliche Salze.

2. Verbindungen nach Anspruch 1, dadurch gekennzeichnet, daß X Disubstitution mit zwei gleichen Substituenten darstellt.

3. Verbindungen nach Anspruch 2, dadurch gekennzeichnet, daß X Dihalogen-, Dimethyl-, Dimethoxy- oder Methylendioxysubstitution darstellt.

4. Verbindungen nach Anspruch 1, dadurch gekennzeichnet, daß X Monosubstitution in der meta- oder para- Stellung darstellt.

5. Verbindung nach Anspruch 3, dadurch gekennzeichnet, daß X für den 3,4-Methylendioxyrest steht.

6. Verbindung nach Anspruch 3, dadurch gekennzeichnet, daß X für Methylreste in 2- und 6-Stellung steht.

7. Verbindung nach Anspruch 3, dadurch gekennzeichnet, daß X für Chloratome in 3- und 4-Stellung steht.

8. Verbindung nach Anspruch 4, dadurch gekennzeichnet, daß X für einen para-Methoxy- oder meta-Chlorrest steht.

9. Verfahren für die Herstellung einer Verbindung nach Anspruch 1, dadurch gekennzeichnet, daß man eine Verbindung der Formel:

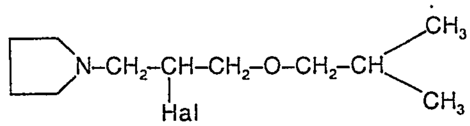

[A]

oder eines von deren Säureadditionssalzen mit einer Verbindung der Formel:

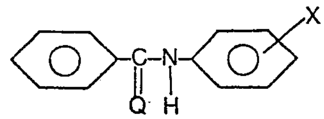

[B]

oder einem von deren Säureadditionssalzen umsetzt, worin X die oben angegebene Bedeutung hat und Q entweder für Sauerstoff oder für zwei Wasserstoffatome steht, und daß man anschließend – wenn Q für Sauerstoff steht – die Verbindung an der Carbonylfunktion reduziert und sie gegebenenfalls in ein pharmazeutisch unbedenkliches Säureadditionssalz umwandelt.

10. Verfahren nach Anspruch 9, dadurch gekennzeichnet, daß man die Reaktion zwischen [A] und [B] durch vorherige Durchführung einer Metallierung der Verbindung der Formel [B] mit einem Alkalimetallhydrid oder -amid erleichtert.

11. Pharmazeutische Zusammensetzung zur Verwendung als Arzneimittel gegen Herz- und Gefäßerkrankungen, insbesondere Angina pectoris, hohen Blutdruck und Rythmusstörungen, dadurch gekennzeichnet, daß sie als Wirkstoff mindestens eine der Verbindungen nach einem der Ansprüche 1 bis 8 zusammen mit geeigneten Trägern enthält.

12. Pharmazeutische Zusammensetzung nach Anspruch 11, dadurch gekennzeichnet, daß sie eine Wirkstoffdosis enthält, die eine tägliche Verabreichung zwischen 1 und 15 mg pro kg Körpergewicht gestattet.